# EUROPEAN PATENT APPLICATION

(11) **EP 1 669 758 A1**
(43) Date of publication of application: **14.06.2006**
(21) Application number: 04028390.5
(22) Date of filing: 01.12.2004
(51) Int. Cl.: G01N 33/558, G01N 33/76, G01N 33/569

(54) **Lateral-flow test device providing improved test result validity**

(71) Applicant: The Jordanian Pharmaceutical Manufacturing Co., 11710 Naor (JO)
(72) Inventor: Mohammed, Murshed AbdelQader, Amman 11118 (JO)
(74) Representative: Winkler, Andreas Fritz Ernst

(57) **Abstract**

The present invention refers to a lateral-flow test device providing improved test result validity comprising at least one indication means indicating whether a test result will be inconclusive and/or at least one indication means indicating that a test result is ready for read. It is further referred to a use of such lateral-flow test device and to a procedure for its manufacture.

## Description

The present invention refers to a lateral-flow test device providing improved test result validity comprising at least one indication means indicating whether a test result will be inconclusive and/or at least one indication means indicating that a test result is ready for read. The present invention further refers to a use of such a lateral-flow test device and to a procedure for manufacture.

### Background of the invention

In recent years the *in vitro* diagnostics (IVD) industry has made enormous efforts to develop membrane-based lateral-flow tests. Such tests have found applications in both clinical and non-clinical fields (ref. 1). A clinical utility of this test format has been shown for more than 150 different analytes, and many of them are target now of commercially available diagnostic products (ref. 3). The wide range of applications for such devices has been reviewed (refs. 1, 2).

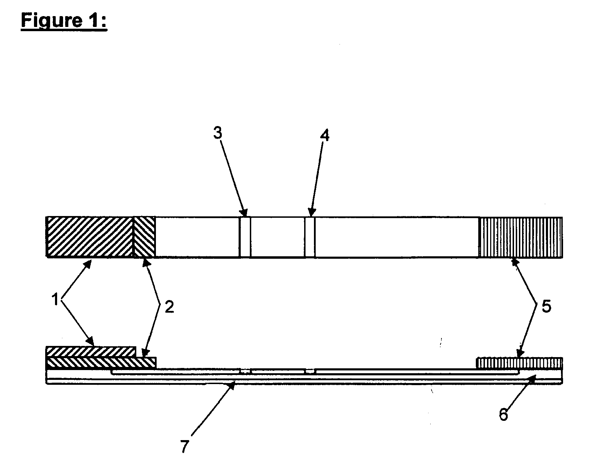

Membrane-based lateral-flow test devices, e.g. rapid-flow immunochromatographic test devices, are made up of a number of components commonly including a sample pad, a conjugate pad, a membrane that incorporates capture reagents, and an absorbent pad (see *Figure 1).* In practice, the user dispenses a patient sample (usually urine or whole blood) onto the sample pad. The sample then flows through the sample pad into the conjugate pad where it mixes with and releases the detector reagent. This mixture then flows across the membrane where it binds with the test and control reagents. When the mixture binds to the reagent that forms the test line, a positive result is indicated. The color intensity of the test line is proportional to the concentration of analyte in the sample. Excess sample that flows beyond the test and control lines is taken up in the absorbent pad.

Membrane-based lateral-flow test devices for diagnostic purposes are easy to operate and thus do not only contribute to the comfort of professional users, e.g. medical stuff, but also allow the operation by non-professionals users, e.g. most patients.

However, one serious problem associated with the use of membrane-based lateral-flow test devices is atmospheric humidity. Most of the rapid test components are extremely hygroscopic, especially nitrocellulose membrane which absorbs moisture strongly and equilibrates with ambient conditions within seconds. As a consequence, false positive (or negative) results may be produced. Thus, unfavorable storage and/or use conditions due to high relative humidity potentially affects the validity of the test result. Therefore, it would be of advantage to have a lateral-flow test device available indicating whether the test result will be inconclusive.

Another problem is that the user must know when the analysis reaction indicating the presence or absence of an analyte is completed and thus the result is ready for read. In all currently available rapid-flow immunochromatographic test devices in the IVD market around the globe, the user is provided with instructions, i.e. by the package insert, such as: "Please read results after x minutes". Thus, the final consumer will be forced to use a timer or a clock, and the need of additional equipment may be more or less uncomfortable. A more serious problem, however, is the risk of inaccurate determination of time since such external time measurement does not have any relationship with the real analysis reaction running at the test device. Reading of result at a time when the analysis reaction is not yet completed or even when the reaction products are already disintegrating potentially produce false results. Therefore, it would be of advantage to have a lateral-flow test device available comprising an internal control with respect to the reading time.

Thus, it is an object of the present invention to provide a lateral-flow test device yielding test results with improved validity.

### Summary of the invention

The object of the present invention is solved by a lateral-flow test device comprising indication means wherein at least one indication means (101) indicates whether a test result will be conclusive.

The object of the present invention is also solved by a lateral-flow test device comprising indication means wherein at least one indication means (102) indicates that a test result is ready for read.

The object of the present invention is also solved by a lateral flow test device comprising indication means wherein at least one indication means (101) indicates whether a test result will be conclusive, and at least one indication means (102) indicates that a test result is ready for read.

In one embodiment of the present invention, the test device further comprises at least one indication means (103) and/or (104).

In one embodiment of the present invention, the test device is a rapid-flow immunochromatographic test device.

In one embodiment of the present invention, the indication means (101) indicates whether the test result will be inconclusive due to humidity.

In one embodiment of the present invention, the indication means (101) and (102) are realized by one single indication means.

In one embodiment of the present invention, the indication means (101) and/or (102) comprise at least one humidity indicator material changing color upon contact with water molecules.

In one embodiment of the present invention, the indication means (101) and/or (102) additionally comprise at least one additive intensifying color change as a component of the humidity indication material.

In one embodiment of the present invention, the humidity indicator material changes color when relative humidity of the atmosphere exceeds 30%, 40%, 50%, and /or 60%.

In one preferred embodiment of the present invention, the humidity indicator material changes color when relative humidity of the atmosphere exceeds 40%.

In one embodiment of the present invention, the humidity indicator material changes color within the first hour after being exposed to relative humidity of more than 30%, 40%, 50%, and/or 60%.

In one preferred embodiment of the present invention, the humidity indicator material changes color within the first hour after being exposed to relative humidity of more than 40%.

In one embodiment of the present invention, the humidity indicator material is selected from the group comprising cobalt chloride, copper chloride, lead iodide and/or other indicating compounds.

In one embodiment of the present invention, the test device comprises indication means for detection and quantification of at least one analyte selected from the group comprising human chorionic gonadotropin (hCG) antigen, *Helcicobacter pylori* antibody, HIV antibody, human lutropin (hLH), human follitropin (hFSH), C-reactive protein, thyroid stimulating hormone (TSH), influenza A virus, influenza B virus, adenovirus, rotavirus, creatine kinase MB isoenzyme (CK-MB), myoglobin, *Chlamydia,* malaria pathogen, prostate-specific antigen (PSA), alpha-fetoprotein (AFP), carcinoembryonic antigen (CEA), tuberculosis (TB) pathogen, *Brucella,* troponin C, troponin I, troponin T, *Toxoplasma,* rubella virus, *Salmonella,* Pan *Salmonella, Listeria,* marihuana, methamphetamine, morphine, amphetamine, cocaine, phencyclidine, barbital, methadone, oxycodone, ethadone, etc.

In one embodiment of the present invention, the analyte is present in bodily fluid selected from the group comprising urine, whole blood, serum, plasma, sperm, menstrual fluid, amniotic fluid, spinal fluid, and synovial fluid.

In one embodiment of the present invention, the analyte is present in solutions, suspensions or dispersions selected from the group comprising cell culture supernatant, cellular extract and tissue extract.

In one embodiment of the present invention, the analyte is present in synthetic solutions and extracts selected from the group comprising environmentral samples, natural and indusrial products.

In one embodiment of the present invention, the test device is in the form of a test strip.

The object of the present invention is further solved by a use of a lateral-flow test device for detection and quantification of at least one analyte, the test device comprising indication means wherein at least one indication means (101) indicates whether a test result will be inconclusive.

The object of the present invention is further solved by a use of a lateral-flow test device for detection and quantification of at least one analyte, the test device comprising indication means wherein at least one indication means (102) indicates that a test result is ready to read.

The object of the present invention is further solved by a use of a lateral flow test device for detection and quantification of at least one analyte, the test device comprising indication means wherein at least one indication means (101) indicates whether a test result will be inconclusive, and at least one indication means (102) indicates that a test result is ready to be read.

In one embodiment of the present invention, the use is for detection and quantification of at least one analyte selected from the group comprising human chorionic gonadotropin (hCG) antigen, *Helcicobacter pylori* antibody, HIV antibody, human lutropin (hLH), human follitropin (hFSH), C-reactive protein, thyroid stimulating hormone (TSH), influenza A virus, influenza B virus, adenovirus, rotavirus, creatine kinase MB isoenzyme (CK-MB), myoglobin, *Chlamydia,* malaria pathogen, prostate-specific antigen (PSA), alpha-fetoprotein (AFP), carcinoembryonic antigen (CEA), tuberculosis (TB) pathogen, *Brucella,* troponin C, troponin I, troponin T, *Toxoplasma,* rubella virus, *Salmonella,* Pan *Salmonella, Listeria,* marihuana, methamphetamine, morphine, amphetamine, cocaine, phencyclidine, barbital, methadone, oxycodone, ethadone, etc.

The object of the present invention is further solved by a procedure of manufacture of a lateral-flow test device comprising the steps of:
(1) preparing at least one humidity indication material;
(2) providing indication means (101) and/or (102) with said humidity indication material.

In one embodiment of the present invention, the humidity indication material used in the procedure of manufacture is selected from the group comprising cobalt chloride, copper chloride and lead iodide compounds.

In one embodiment of the present invention, the procedure of manufacture additionally comprises the steps of:
(1) Preparing at least one gold releasing pad, comprising the steps of:
   (a) preparing a 1% gold chloride solution;
   (b) preparing a 4% trisodium citrate solution and/or another reducing solution according to the colloidal gold solution particle size;
   (c) preparing a colloidal gold solution;
   (d) preparing a 1% potassium carbonate solution;
   (e) preparing a 1% polyethylene glycol solution;
   (f) preparing a stabilizing buffer;
   (g) preparing a borate buffer;
   (h) preparing a sucrose solution.;
   (i) conjugating a specific antibody or antigen with the colloidal gold in solution of step (c);
   (j) soaking a fiber releasing pad into the conjugate solution of step (i) and drying;
   (k) cutting sheets into 8 mm width strips.
(2) Preparing at least one antibody and/or antigen printed nitrocellulose membrane, comprising the steps of.
   (a) preparing a suitable antibody and/or antigen diluting buffer;
   (b) spray printing antibodies and/or antigens onto a nitrocellulose membrane and drying;
   (c) preparing a nitrocellulose membrane blocking solution;
   (d) blocking of the nitrocellulose membrane and drying.
(3) Preparing at least one sample pad, comprising the steps of:
   (a) preparing a suitable sample pad buffer;
   (b) soaking a sample pad into the buffer of step (a) and drying.
(4) Preparing at least one absorbent indication pad, comprising the step of laminating strips of an indication card material onto a pad.
(5) Laminating and cutting, comprising the steps of:
   (a) laminating the antibody and/or antigen printed nitrocellulose membrane onto a vinyl backing;
   (b) laminating a gold conjugate strip;
   (c) laminating an absorbent pad;
   (d) laminating a sample pad;
   (e) cutting a laminated card into strips of suitable width depending on a plastic housing and test format.
(6) Assembling, comprising the steps of:
   (a) assembling the strips of step (5e) and a sample filter into the bottom part of a plastic housing;
   (b) laminating of an atmospheric humidity indication piece onto the inside of the top part of the plastic housing;
   (c) joining the top and bottom parts of the plastic housing;
   (d) joining the cap;
   (e) inserting the test into the pouch with a desiccant and sealing the pouch.

In one alternative embodiment of the present invention, the preparation of an absorbent indication pad of step (4) above alternatively comprises an application a humidity indicator material solution by impregnating the absorbent pad with the appropriate chemical solution or by spraying this solution onto the pad.

The term "indication means" as used herein may refer to means suitable for indicating the detection of an analyte, e.g. by indicating an analysis reaction product. Such indication may be mediated optically or acoustically, via radioactivity or fluorescence.

The term "humidity indicator material" changing color upon contact with water molecules comprises inorganic compounds which have long been known for quantitatively detecting humidity. Such indicator material has found application also in the medical field where changes in relative humidity are of critical importance. It allows visual inspection of the condition of a sealed package by readily detecting any change in the relative humidity. However, further physical or chemical methods for detecting and indicating humidity are considered.

Examples of inorganic compounds serving as humidity indicators are cobalt chloride, copper chloride or lead iodide compounds. Cobalt(II)chloride forms hydrates of different colors ranging between blue-violet (CoCl₂ · H₂O) and pink (CoCl₂ · 6H₂O). Copper chloride changes color from brown to azure in the presence of water molecules. Solutions of KPbI₃ have been used to impregnate filter paper for detecting traces of water since the complex is split in the presence of water leading to gold-colored PbI₂.

For realizing the present invention, the humidity indicator material can be applied in form of a humidity indication spot on special cards and/or by impregnating of the absorbent pad with an appropriate chemical solution and/or spraying of the chemical solution onto the absorbent pad.

The sensitivity of humidity indication may be varied by applying different amounts of humidity indication material and different concentrations of its solution, respectively. Furthermore, the kind of humidity indication material may be selected according to the preferred sensitivity. Also the thickness and location of the special card may be selected with regard to the preferred sensitivity.

The indication that a test result is ready for read can be managed by variations in volume and properties of the sample to be tested. Furthermore, a gold releasing pad may be used and its material, location and length may be varied. When using nitrocellulose membrane, a blocking procedure may be applied. Also variations in the material, location and length of the sample pad, the absorbent pad or the membrane may be employed.

The term "ready for read" refers to the time following the completion of an analysis reaction but prior to disintegration of the reaction products.

### Brief description of the figures

- *Figure 1*: shows top and side views of a typical rapid-flow immunochromatographic test device comprising a sample pad (1), a conjugate pad (2), a capture (test) zone (3), e.g. a membrane that incorporates at least one capture reagent, a control (negative) zone (4), an absorbent pad (5), an adhesive (6) and a plastic backing (7).

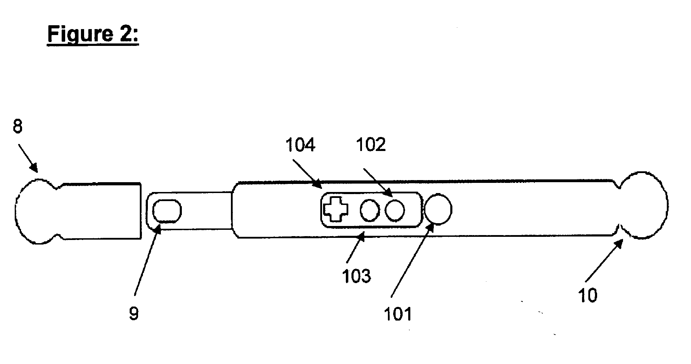

- *Figure 2*: shows a preferred embodiment of a lateral-flow test device of the present invention comprising a removable cap (8), a sample application window (9), a handle (10), and indication means, namely:
an indication means (101) realized by a humidity indication hole;
an indication means (102) realized by a result reading time indication hole;
an indication means (103) realized by a control line region;
an indication means (104) realized by a test line region.
- *Figure 3*: demonstrates the operation of a lateral-flow test device as shown *Figure 2* with indication means (201) and (202) comprising cobalt chloride as the humidity indicator, and further with indication means (203) and (204).
- *Figure 4*: demonstrates the operation of a lateral flow test device as shown *Figure* 2 with indication means (301) and (302) comprising copper chloride as the humidity indicator, and further with indication means (303) and (304).

### Detailed description of the invention

### Examples

### Example 1: Operation of a test device of the present invention using cobalt chloride

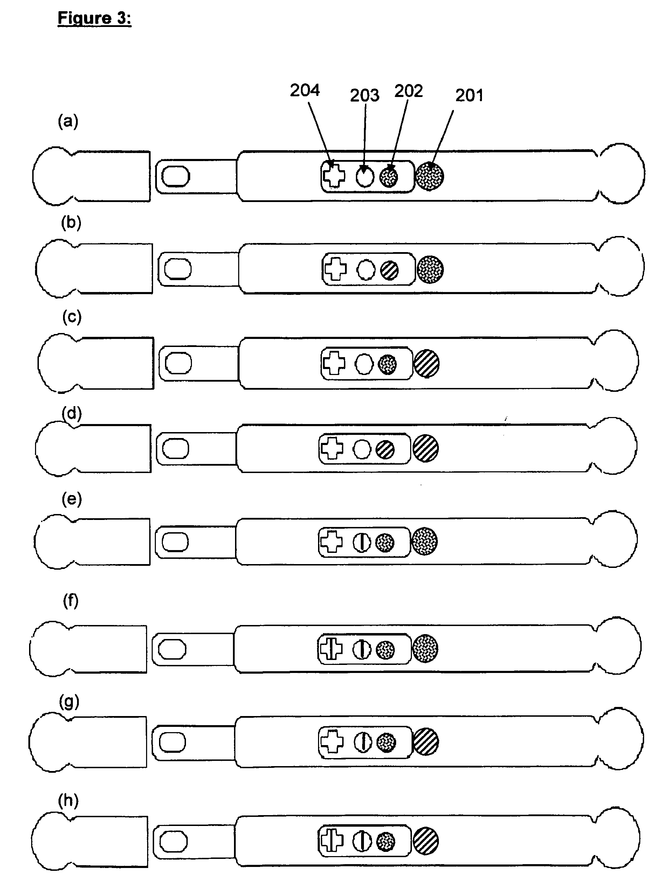

It is referred to *Figure* 3. Both indication means (201) (= humidity indication hole) and indication means (202) (= result reading time indication hole) comprise cobalt chloride as the indicator material, optionally with other additives to meet the intensified color change requirements. Indication means (202) together with indication means (203) (= control line region) and (204) (= test line region) are constructed such that they get in contact with a sample applied to the sample application window. In contrast, means (201) is not intended to physically contact the sample, e.g. by fluid communication. As a consequence, indication means (201) and indication means (202) are capable of indicating high relative atmospheric humidity, e.g. more than 40%, whereas only indication means (202) is capable of indicating that the sample has passed indication means (204) and (203), so that the reaction is completed and thus the test result is ready for read.

The information provided by a cobalt chloride test device as described is drawn up below (see also *Figure* 3, wherein "pink" is represented by a dotted region, "blue" is represented by a diagonally hatched region, and the "pink/purple band" is represented by a black band).

| | | |
|---|---|---|
| (a) | Indication means (201): | pink |
| | Indication means (202): | pink |
| | Result: | **inconclusive** |
| | Comment: | The test must be repeated with new device. |
| (b) | Indication means (201): | pink |
| | Indication means (202): | blue |
| | Result: | **inconclusive** |
| | Comment: | The test must be repeated with new device. |
| (c) | Indication means (201): | blue |
| | Indication means (202): | pink |
| | Result: | inconclusive |
| | Comment: | The test must be repeated with new device. |
| (d) | Indication means (201): | blue |
| | Indication means (202): | blue |
| | Result: | conclusive |
| | Comment: | The test device can be safely used. |
| (e) | Indication means (201): | pink |
| | Indication means (202): | pink |
| | Indication means (203): | pink/purple band |
| | Indication means (204): | blank |
| | Result: | **negative** |
| (f) | Indication means (201): | pink |
| | Indication means (202): | pink |
| | Indication means (203): | pink/purple band |
| | Indication means (204): | pink/purple band |
| | Result: | **positive** |
| (g) | Indication means (201): | blue |
| | Indication means (202): | pink |
| | Indication means (203): | pink/purple band |
| | Indication means (204): | blank |
| | Result: | **negative** |
| (h) | Indication means (201): | blue |
| | Indication means (202): | pink |
| | Indication means (203): | pink/purple band |
| | Indication means (204): | pink/purple band |
| | Result: | **positive** |

### Example 2: Operation of a test device of the present invention using copper chloride.

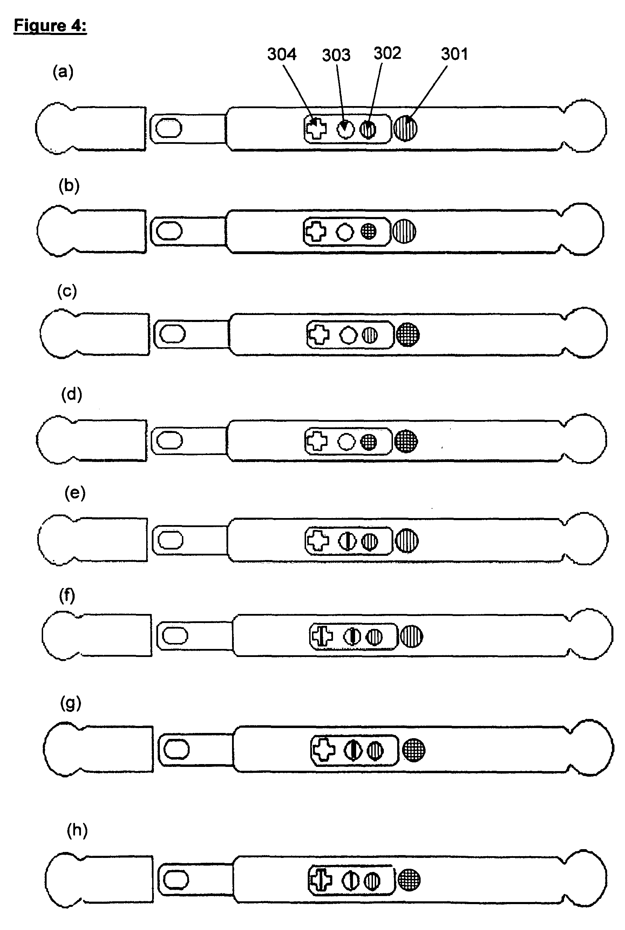

It is referred to *Figure* 4. The indication means are identified as follows: (301) = humidity indication hole; (302) = result reading time indication hole; (303) = control line region; and (304) = test line region. In contrast to the test device of Example 1, this test device comprises copper chloride instead of cobalt chloride as the humidity indicator material.

The information provided by a copper chloride test device as described is drawn up below (see also *Figure* 4, wherein "azure" is represented a vertically hatched region, "brown" is represented by a squared region, and the "pink/purple band" is represented by a black band).

| | | |
|---|---|---|
| (a) | Indication means (301): | azure |
| | Indication means (302): | azure |
| | Result: | **inconclusive** |
| | Comment: | The test must be repeated with new device. |
| (b) | Indication means (301): | azure |
| | Indication means (302): | brown |
| | Result: | **inconclusive** |
| | Comment: | The test must be repeated with new device. |
| (c) | Indication means (301): | brown |
| | Indication means (302): | azure |
| | Result: | **inconclusive** |
| | Comment: | The test must be repeated with new device. |
| (d) | Indication means (301): | brown |
| | Indication means (302): | brown |
| | Result: | conclusive |
| | Comment: | The test device can be safely used. |
| (e) | Indication means (301): | azure |
| | Indication means (302): | azure |
| | Indication means (303): | pink/purple band |
| | Indication means (304): | blank |
| | Result: | negative |
| (f) | Indication means (301): | azure |
| | Indication means (302): | azure |
| | Indication means (303): | pink/purple band |
| | Indication means (304): | pink/purple band |
| | Result: | positive |
| (g) | Indication means (301): | brown |
| | Indication means (302): | azure |
| | Indication means (303): | pink/purple band |
| | Indication means (304): | blank |
| | Result: | negative |
| (h) | Indication means (301): | brown |
| | Indication means (302): | azure |
| | Indication means (303): | pink/purple band |
| | Indication means (304): | pink/purple band |
| | Result: | positive |

### Example 3: Use of a test device of the present invention for hCG antigen detection

Color changes within 1-2 minutes by considering the following parameters:
immerging an absorbent pad with 40% humidity indicator reagent (or a layer of three 40% spots);
high flow rate system components by using low capillary rise of a nitrocellulose membrane, enough sample volume, etc.;
high concentration of printed antibodies.

### Example 4: Use of a test device of the present invention for H. pylori antigen detection

Color changes within 10 minutes by considering the following parameters:
immerging an absorbent pad with 60% humidity indicator reagent (or 40% spot);
low flow rate system components by using a suitable nitrocellulose membrane of high capillary rise, small buffer volume, etc.

### Example 5: Use of a test device of the present invention for HIV antibody detection

Color changes within 15 minutes by considering the following parameters:
immerging an absorbent pad with 60% humidity indicator reagent (or 50% spot);
very slow flow rate system components by using a suitable nitrocellulose membrane of higher capillary rise, smaller buffer volume, etc.

### References

(1) J Chandler, N Robinson, and K Whiting, "Handling False Signals in Gold-Based Rapid Tests" - IVD Technology 7, no. 2 (2001): 34-45;
   http://www.devicelink.com/ivdt/archive/01/03/002.html
(2) J Chandler, T Gurmin, and N Robinson, "The Place of Gold in Rapid Tests" - IVD Technology 6, no. 2 (2000): 37-49;
   http://www.devicelink.com/ivdt/archive/00/03/004.html
(3) TC Tisone et al., "Image Analysis for Rapid-Flow Diagnostics" - IVD Technology 5, no. 5 (1999): 52-58;
   http://www.devicelink.com/ivdt/archive/99/09/010.html

## Claims

1. A lateral-flow test device comprising indication means
- **characterized in that** -
at least one indication means (101) indicates whether a test result will be inconclusive.

2. A lateral-flow test device comprising indication means
- **characterized in that** -
at least one indication means (102) indicates that a test result is ready for read.

3. A lateral-flow test device comprising indication means
- **characterized in that** -
at least one indication means (101) indicates whether a test result will be inconclusive, and at least one indication means (102) indicates that a test result is ready for read.

4. The test device according to any of claims 1-3, wherein the test device is a rapid-flow immunochromatographic test device.

5. The test device according to claim 1 or 3, wherein the indication means (101) indicates whether the validity of the test result will be inconclusive due to humidity.

6. The device according to claim 3, wherein the indication means (101) and (102) are realized by one single indication means.

7. The test device according to any of the preceding claims, wherein the indication means (101) and/or (102) comprise at least one humidity indicator material changing color upon contact with water molecules.

8. The test device according to any of the preceding claims, wherein the indication means (101) and/or (102) additionally comprise at least one additive intensifying color change.

9. The test device according to any of the preceding claims, wherein the humidity indicator material changes color when relative humidity of the atmosphere exceeds 40%.

10. The test device according to claim 9, wherein the humidity indicator material changes color within the first hour after being exposed to relative humidity of more than 40%.

11. The test device according to any of the preceding claims, wherein the humidity indicator material is selected from the group comprising cobalt chloride, copper chloride and lead iodide compounds.

12. The test device according to any of the preceding claims, wherein the test device comprises indication means for detection and quantification of at least one analyte selected from the group comprising hCG antigen, *Helicobacter pylori* antibody and HIV antibody.

13. The test device according to any of the preceding claims, wherein the analyte is present in bodily fluid selected from the group comprising urine, whole blood, serum, plasma, sperm, menstrual fluid, amniotic fluid, spinal fluid and synovial fluid

14. The test device according to any of the preceding claims, wherein the analyte is present in solutions, suspensions or dispersions selected from the group comprising cell culture supernatant, cellular extract and tissue extract.

15. The test device according to any of the preceding claims, wherein the test device is in the form of a test strip.

16. A use of a lateral-flow test device for detection and quantification of at least one analyte, the test device comprising indication means
- **characterized in that** -
at least one indication means (101) indicates whether a test result will be inconclusive.

17. A use of a lateral-flow test device for detection and quantification of at least one analyte, the test device comprising indication means
- **characterized in that** -
at least one indication means (102) indicates that a test result is ready for read.

18. A use of a lateral-flow test device for detection and quantification of at least one analyte, the test device comprising indication means
- **characterized in that** -
at least one indication means (101) indicates whether a test result will be inconclusive, and at least one indication means (102) indicates that a test result is ready for read.

19. The use according to any of claims 16-18 for detection and quantification of at least one analyte selected from the group comprising hCG antigen, *Helicobacter pylori* antibody and HIV antibody.

20. A procedure of manufacture of a lateral flow test device comprising the steps of:
(1) preparing at least one humidity indication material;
(2) providing indication means (101) and/or (102) with said humidity indication material.

21. The procedure according to claim 20, wherein the humidity indication material is selected from the group comprising cobalt chloride, copper chloride and lead iodide compounds.
